# EUROPEAN PATENT APPLICATION

(11) **EP 4 604 128 A1**
(43) Date of publication of application: **20.08.2025**
(21) Application number: 23901159.6
(22) Date of filing: 08.12.2023
(51) Int. Cl.: G16B 40/20, G06N 3/04, G16B 30/10, G16B 25/10

(54) **DATA AUGMENTATION METHOD, DEVICE, AND PROGRAM FOR MHC CLASS II BINDING AND IMMUNOGENICITY PREDICTION MODEL**

(30) Priority: 09.12.2022 KR 20220171801
(71) Applicant: LG Management Development Institute Co., Ltd., Seoul 07336 (KR)
(72) Inventor: KIM, Kiyoung, Seoul 07811 (KR); HWANG, Doyeong, Seoul 07796 (KR); HORMAZABAL, Rodrigo, Seoul 07372 (KR); HAN, Sehui, Seoul 07665 (KR); LEE, Honglak, Seoul 07789 (KR)
(74) Representative: BCKIP Part mbB
(86) International application number: PCT/KR2023/020221
(87) International publication number: WO 2024/123142

(57) **Abstract**

Data augmentation methods, devices, and programs for an MHC class II binding and immunogenicity predictive models are disclosed. A data augmentation device according to one embodiment of the present invention may include a processor that is implemented to select a plurality of augmentation target data including first-type data and second-type data to be augmented according to a predetermined selection condition from the original data, to augment the selected plurality of augmentation target data according to a predetermined augmentation condition, wherein the first-type data and the second-type data are respectively augmented according to an augmentation condition of the first-type data and an augmentation condition of the second-type data to generate a plurality of augmentation data, and to modify labeling of the plurality of augmentation data, wherein labels are modified according to different labeling conditions for the first-type data and the second-type data.

## Description

### [Technical Field]

The present disclosure relates to a data augmentation method and device for augmentation of training data. More specifically, the present disclosure relates to a data augmentation method, device, and program for an MHC Class II binding and immunogenicity predictive models.

### [Background Art]

Recently, various concepts and learning models have been developed in the field of artificial intelligence technology, and research on data prediction using the same has been actively conducted.

However, when predicting data based on an artificial intelligence-based neural network, there is a growing need to develop training or prediction algorithm for the learning model to derive results with a high prediction probability.

In addition, in order to improve the confidence of data prediction results, measures to input a larger number of data are being sought.

### [Disclosure]

### [Technical Problem]

The present embodiments disclosed in the present disclosure are directed to providing data augmentation methods, devices, and programs for an MHC class II binding and immunogenicity predictive models to augment data to be input during artificial intelligence-based predictive training.

Objects of the present disclosure are not limited to the above-described object, and other objects that are not mentioned will be clearly understood by those skilled in the art from the following description.

### [Technical Solution]

A data augmentation device according to an aspect of the present disclosure for achieving the objects may include a memory, and a processor configured to communicate with the memory and implement augmentation of original data to be trained, wherein the processor may be implemented to select a plurality of augmentation target data including first-type data and second-type data to be augmented according to a predetermined selection condition from the original data, augment the selected plurality of augmentation target data according to a predetermined augmentation condition, wherein the first-type data and the second-type data are respectively augmented according to an augmentation condition of the first-type data and an augmentation condition of the second-type data to generate a plurality of augmentation data, and modify labeling of the plurality of augmentation data, wherein labels are modified according to different labeling conditions for the first-type data and the second-type data, and the original data may be a peptide feature for binding of a major histocompatibility complex (MHC) class II feature.

In addition, when selecting the plurality of augmentation target data, the processor may select the first-type data including at least one positive data matching a first selection condition among the original data, wherein the first selection condition may be a condition in which an IC50 label is less than a predetermined concentration value and a peptide length is less than or equal to a predetermined number.

In addition, when selecting the plurality of augmentation target data, the processor may select the second-type data including at least one negative data matching a second selection condition among the original data, wherein the second selection condition may be a condition in which an IC50 label is greater than a predetermined concentration value and a peptide length is greater than or equal to a predetermined number.

In addition, when generating the plurality of augmentation data, the processor may randomly add all amino acids to each of the plurality of augmentation target data, wherein a randomly selected amino acid sequence may be added to an N-terminus of a peptide original sequence of the first-type data as one sequence, a randomly selected amino acid sequence may be added to a C-terminus of the peptide original sequence of the first-type data as one sequence, and a randomly selected amino acid sequence may be added to each of the N-terminus and C-terminus of the peptide original sequence of the first-type data as one sequence, to augment the first-type data of the plurality of augmentation data.

In addition, when generating the plurality of augmentation data, the processor may add a sequence to each of the plurality of augmentation target data using an amino acid sequence pattern of a human protein, wherein one sequence may be added to an N-terminus of a peptide original sequence of the first-type data using the amino acid sequence pattern of the human protein, one sequence may be added to a C-terminus of the peptide original sequence of the first-type data using the amino acid sequence pattern of the human protein, and one sequence may be added to each of the N-terminus and C-terminus of the peptide original sequence of the first-type data using the amino acid sequence pattern of the human protein, to augment the first-type data of the plurality of augmentation data.

In addition, when generating the plurality of augmentation data, the processor may remove sequences from both termini of a peptide original sequence of the second-type data in the plurality of augmentation target data until a length of the peptide original sequence matches a predetermined number of sequences.

In addition, when modifying the labeling of the plurality of augmentation data, the processor may normalize a label of the corresponding original data of each of the plurality of augmentation data, and obtain a final pseudo label according to the labeling conditions for the first-type data and the second-type data, wherein the final pseudo label may be calculated using a predetermined label constant value based on the normalized pseudo label of the original data and a binding affinity of a peptide to an MHC class II molecule.

The processor may delete duplicate data by comparing the plurality of augmentation data with the original data.

In addition, a data augmentation method according to another aspect of the present disclosure, in the method performed by a computer device, may include selecting a plurality of augmentation target data including first-type data and second-type data to be augmented according to a predetermined selection condition from original data, augmenting the selected plurality of augmentation target data according to a predetermined augmentation condition, wherein the first-type data and the second-type data are respectively augmented according to an augmentation condition of the first-type data and an augmentation condition of the second-type data to generate a plurality of augmentation data, and modifying labeling of the plurality of augmentation data, wherein labels are modified according to different labeling conditions for the first-type data and the second-type data, wherein the original data may be a peptide feature for binding of a major histocompatibility complex (MHC) class II feature.

In addition, when selecting the plurality of augmentation target data, the data augmentation method may select the first-type data including at least one positive data matching a first selection condition among the original data, wherein the first selection condition may be a condition in which an IC50 label is less than a predetermined concentration value and a peptide length is less than or equal to a predetermined number.

In addition, when selecting the plurality of augmentation target data, the data augmentation method may select the second-type data including at least one negative data matching a second selection condition among the original data, wherein the second selection condition may be a condition in which an IC50 label is greater than a predetermined concentration value and a peptide length is greater than or equal to a predetermined number.

In addition, when generating the plurality of augmentation data, the data augmentation method may randomly add all amino acids to each of the plurality of augmentation target data, wherein a randomly selected amino acid sequence may be added to an N-terminus of a peptide original sequence of the first-type data as one sequence, a randomly selected amino acid sequence may be added to a C-terminus of the peptide original sequence of the first-type data as one sequence, and a randomly selected amino acid sequence may be added to each of the N-terminus and C-terminus of the peptide original sequence of the first-type data as one sequence, to augment the first-type data of the plurality of augmentation data.

In addition, when generating the plurality of augmentation data, the data augmentation method may add a sequence to each of the plurality of augmentation target data using an amino acid sequence pattern of a human protein, wherein one sequence may be added to an N-terminus of a peptide original sequence of the first-type data using the amino acid sequence pattern of the human protein, one sequence may be added to a C-terminus of the peptide original sequence of the first-type data using the amino acid sequence pattern of the human protein, and one sequence may be added to each of the N-terminus and C-terminus of the peptide original sequence of the first-type data using the amino acid sequence pattern of the human protein, to augment the first-type data of the plurality of augmentation data.

In addition, when generating the plurality of augmentation data, the data augmentation method may remove sequences from both termini of a peptide original sequence of the second-type data in the plurality of augmentation target data until a length of the peptide original sequence matches a predetermined number of sequences.

In addition, when modifying the labeling of the plurality of augmentation data, the data augmentation method may normalize a label of the corresponding original data of each of the plurality of augmentation data, and may obtain a final pseudo label according to the labeling conditions for the first-type data and the second-type data, wherein the final pseudo label may be calculated using a predetermined label constant value based on the normalized pseudo label of the original data and a binding affinity of a peptide to an MHC class II molecule.

After modifying labeling of the plurality of augmentation data, the data augmentation method may delete duplicate data by comparing the plurality of augmentation data with the original data.

In addition, a computer program stored in a computer-readable recording medium for executing the method for implementing the present disclosure may be further provided.

In addition, a computer-readable recording medium recording a computer program for executing the method for implementing the present disclosure may be further provided.

### [Advantageous Effects]

According to the above-described technical solution of the present disclosure, since input data of a learning model for predicting MHC class II binding and immunogenicity is augmented, which is performed by selecting augmentation target data based on various conditions including an IC50 label, the quality of the augmented data can be improved and the confidence of the binding and immunogenicity prediction results trained based on the augmented data can be improved.

Effects of the present disclosure are not limited to the above effects, and other effects that are not mentioned will be clearly understood by those skilled in the art from the following description.

### [Description of Drawings]

FIG. 1A is a view showing a structure of an MHC class II according to the present disclosure.
FIG. 1B is an exemplary diagram for briefly describing a data augmentation method according to the present disclosure.
FIG. 1C is a diagram showing an overall structure of a learning model according to the present disclosure.
FIG. 2 is a diagram showing a configuration of a computer device according to the present disclosure.
FIG. 3 is an exemplary diagram for describing a method of selecting augmentation target data according to the present disclosure.
FIGS. 4 to 6 are exemplary diagrams for describing a method of augmenting the augmentation target data according to the present disclosure.
FIG. 7 is an exemplary diagram for describing a pseudo labeling method according to the present disclosure.
FIG. 8 is a flowchart for describing a data augmentation method according to the present disclosure.
FIGS. 9 and 10 are flowcharts for describing the data augmentation method of FIG. 8 in detail.

### [Modes of the Invention]

The same reference numerals refer to the same components throughout the present disclosure. The present disclosure does not describe all elements of the embodiments, and common content in the art to which the present disclosure pertains or content that overlaps between the embodiments will be is omitted. Terms "unit," "module," "member," and "block" used in the specification may be implemented as software or hardware, and according to the embodiments, a plurality of "units," "modules," "members," and "blocks" may be implemented as one component, or one "unit," "module," "member," and "block" may also include a plurality of components.

Throughout the specification, when a first component is described as being "connected" to a second component, this includes not only a case in which the first component is directly connected to the second component but also a case in which the first component is indirectly connected to the second component, and the indirect connection includes connection through a wireless communication network.

In addition, when a certain portion is described as "including" a certain component, it means further including another component rather than precluding another component unless specifically stated otherwise.

Throughout the present specification, when a first member is described as being positioned "on" a second member, this includes both a case in which the first member is in contact with the second member and a case in which a third member is present between the two members.

Terms such as first and second are used to distinguish one component from another, and the components are not limited by the above-described terms.

A singular expression includes plural expressions unless the context clearly dictates otherwise.

In each operation, identification symbols are used for convenience of description, and the identification symbols do not describe the sequence of each operation, and each operation may be performed in a different sequence from the specified sequence unless a specific sequence is clearly described in context.

Hereinafter, the operation principles and embodiments of the present disclosure will be described with reference to the accompanying drawings.

A "data augmentation device according to the present disclosure" in the present specification includes all types of devices that can perform computational processing and provide results to a user. For example, the data augmentation device according to the present disclosure may include all of a computer, a server device, and a portable terminal, or may be in the form of any one of them.

Here, the computer may include, for example, a notebook, a desktop, a laptop, a tablet PC, a slate PC, etc., which are equipped with a web browser.

The server device is a server that processes information in communication with an external device, and may include an application server, a computing server, a database server, a file server, a game server, a mail server, a proxy server, and a web server.

The portable terminal is, for example, a wireless communication device ensuring portability and mobility and may include all kinds of handheld-based wireless communication devices such as a personal communication system (PCS), a global system for mobile communications (GSM), a personal digital cellular (PDC), a personal handyphone system (PHS), a personal digital assistant (PDA), international mobile telecommunication-2000 (IMT-2000), code division multiple access-2000 (CDMA-2000), w-code division multiple access (W-CDMA), a wireless broadband internet (WiBro) terminal, a smart phone, and wearable devices such as a watch, a ring, a bracelet, an anklet, a necklace, glasses, contact lenses, or a head-mounted device (HMD).

" Antigen " in the present disclosure, may be a substance that induces an immune response.

A neoantigen may refer to a novel protein formed in a cancer cell when a specific mutation occurs in tumor DNA. The neoantigen is characterized by being generated by the mutation and being expressed only in the cancer cell. The neoantigen may include a polypeptide sequence or a nucleotide sequence. The mutation may include a frameshift or non-lattice shift indel, a missense or nonsense substitution, a splice site alteration, a genomic rearrangement or gene fusion, or any genomic or expression alteration causing a new ORF. The mutation may also include a splice variant. A post-translational modification specific to a tumor cell may include an abnormal phosphorylation. The post-translational modification specific to the tumor cell may also include a proteasome-generated spliced antigen.

"Epitope" in the present disclosure may refer to a specific portion of an antigen to which an antibody or a T-cell receptor normally binds.

"Major histocompatibility complex(MHC)" in the present disclosure may be a protein that presents a 'peptide' synthesized in a specific cell on a surface of the cell, thereby enabling a T-cell to identify the cell.

"Peptide" in the present disclosure is a polymer of amino acids. For convenience of explanation, hereinafter, the "peptide" may refer to an amino acid polymer or an amino acid sequence that is expressed on a surface of the cancer cell.

"MHC class II" in the present disclosure may refer to a protein that is expressed on an antigen-presenting cell and activates a Helper T cell, thereby regulating various immune responses.

"MHC class II-peptide complex" in the present disclosure may refer to a complex structure formed by the MHC class II and the peptide, which is expressed on a surface of the antigen-presenting cell or the cancer cell. The Helper T-cell may recognize the MHC class II-peptide complex and perform the immune response.

The cancer cell may generate the neoantigen. The MHC Class II may be primarily expressed on the antigen-presenting cell. The antigen-presenting cell may degrade the neoantigen generated in a cancer, and the epitope derived from the neoantigen may be presented on the surface by the MHC class II. The Helper T cell recognizes the MHC class II-epitope and triggers an immune response. Accordingly, it is necessary to predict a MHC-peptide binding in order to identify the neoantigen generated by the cancer cell.

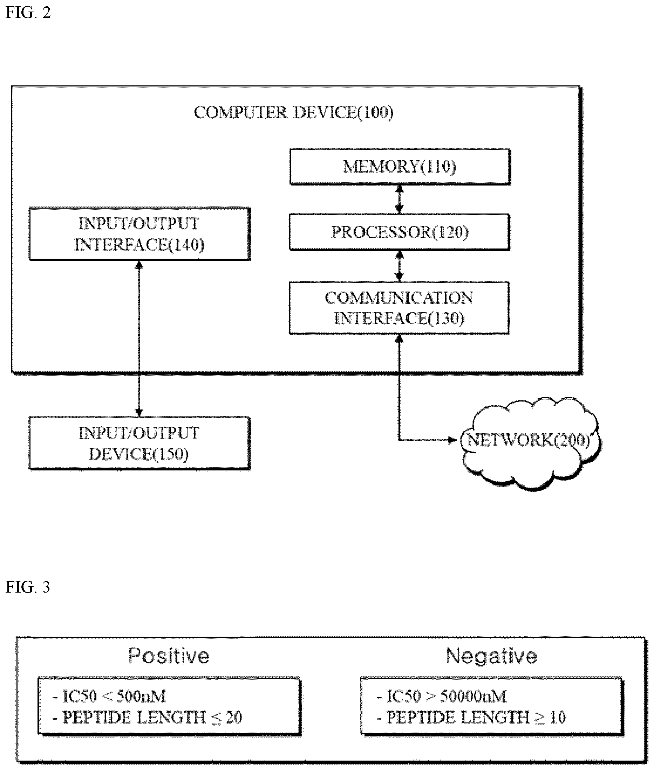

The present disclosure is intended to augment data input into a learning model that predicts whether the MHC class II is bound to a peptide sequence and the activation of the T-cell based on a sequence transformation neural network implemented through training. A series of operations or algorithms for this purpose may be performed by a computer device, and the detailed configuration of the computer device will be described based on FIG. 2 described below. In the present disclosure, the computer device may refer to a data augmentation device.

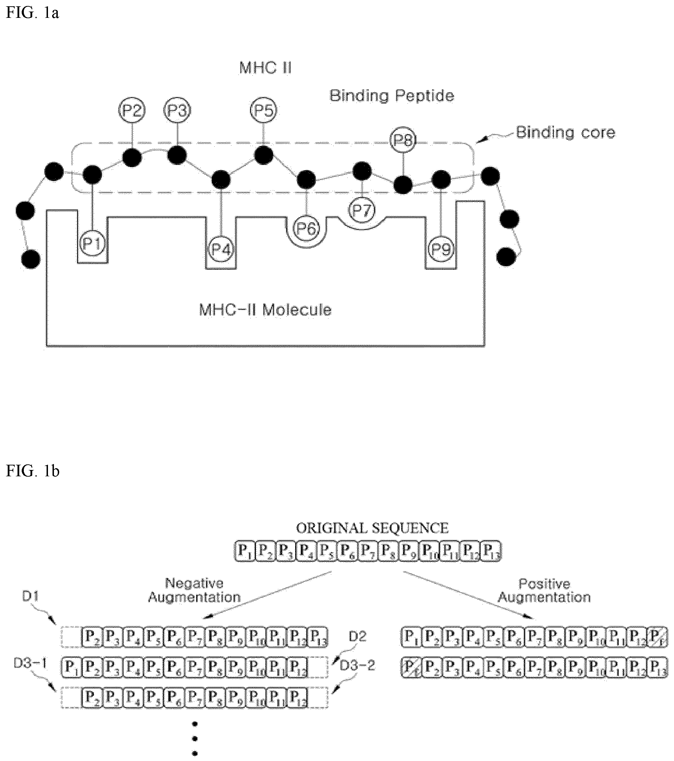

FIG. 1A is a view showing a structure of an MHC class II according to the present disclosure, and FIG. 1B is an exemplary diagram for briefly describing a data augmentation method according to the present disclosure.

The MHC may be a group of cell surface molecules that serve as a biochemical marker distinguishing individuals, and may serve as a mediator to recognize a target substance in an immune response as an antigen.

The MHC may be classified into MHC class I and MHC class II groups based on molecular forms, and the difficulty of the prediction may vary due to differences in antigen binding sites.

The MHC class II may have binding sites composed of different substances, and may bind to peptides of 13 to 17 amino acids.

As shown in FIG. 1A, unlike MHC class I, the MHC class II may consist of two chains of an α chain and a β chain, and may be formed in a structure in which both ends are open.

Due to the open structure at both ends of the MHC class II, when the peptide includes a binding core sequence, the binding may be possible even when another sequence is added at both ends. In addition, when the peptide does not include the binding core sequence, the peptide does not bind to MHC class II even when sequences at both end are removed.

Referring to FIG. 1B, the present disclosure may process data augmentation by adding or deleting sequences in an original sequence of the peptide based on the open structure of the MHC class II. Specifically, in positive augmentation, one sequence (Pᵣ) may be added to each of both ends P₁₃ and P₁ of the original sequence. In addition, in negative augmentation, n sequences D1, D2, D3-1, and D3-2 may be removed from each of both ends P₁₃ and P₁ of the original sequence to augment the data. In this case, a final sequence length of the augmented data may be limited to 10 or more.

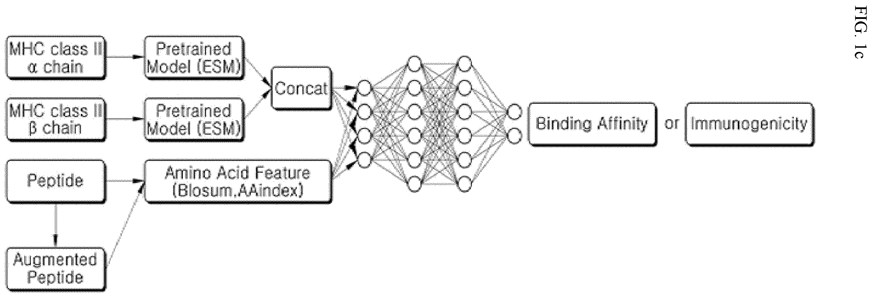

FIG. 1C is a diagram showing an overall structure of a learning model according to the present disclosure.

Referring to FIG. 1C, the learning model based on a sequence transformation neural network (NN) according to the present disclosure may receive a MHC class II α chain feature and a MHC class II β chain feature as first input data and a peptide feature and an augmented peptide feature as second input data.

The first input data may determine a first key and a first value for the first input data through predetermined pre-training based on the MHC class II α chain feature and the MHC class II β chain feature, and generate a first query for the first input data through a multi-head self attention operation based on the second input data corresponding to the first input data.

Based on the above-described first key, first value, and first query, a scaled dot product attention operation may be performed, and by concatenating each attention head, a matrix in which each sequence is converted into a vector may be output as the first input data for training.

The second input data may include the peptide feature (sequences) and the augmented peptide feature, and may be an amino acid feature using both an amino acid substitution matrix (BLOSUM) and a physicochemical property (AAindex).

When receiving the first input data and the second input data, the learning model may learn a MHC class II binding affinity and immunogenicity according to the first input data and the second input data. In this case, the MHC class II binding affinity may mean the possibility of binding between the peptide sequence and the MHC class II, and the immunogenicity may mean whether T-cell activation occurs.

Through the above-described process, a sequence transformation neural network (NN) for transforming an input sequence may be implemented.

FIG. 2 is a diagram showing a configuration of a computer device according to the present disclosure.

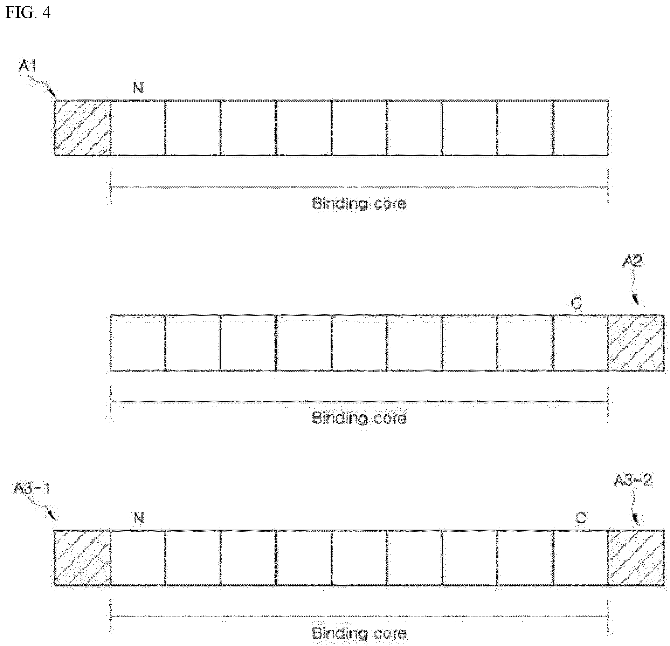

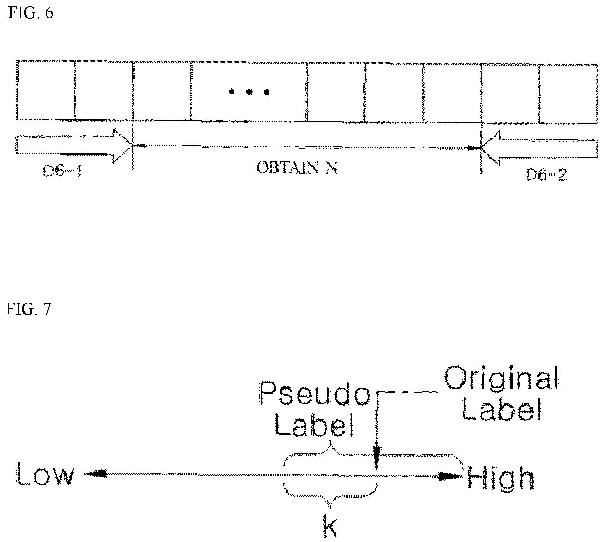

Hereinafter, descriptions will be made with reference to FIG. 3, which is an exemplary diagram for describing a method of selecting augmentation target data according to the present disclosure, FIGS. 4 to 6, which are exemplary diagrams for describing a method of augmenting the augmentation target data according to the present disclosure, and FIG. 7, which is an exemplary diagram for describing a pseudo labeling method according to the present disclosure.

Referring to FIG. 2, a computer device 100 may include a memory 110, a processor 120, a communication interface 130, an input/output interface 140, and an input/output device 150. However, each component is shown as one component in FIG 2, but this is only for convenience of explanation, and each component may be provided as at least one component as needed.

The memory 110 may store a computer program for providing the data augmentation method, and the stored computer program may be read and driven by the processor 120. The memory 110 may store any form of information generated or determined by the processor 120 and any form of information received by the communication interface 130.

The memory 110 may store data that supports various functions of the computer device 100 and a program for the operation of the processor 120, store input/output data (e.g., the original data, the augmentation target data, augmentation data, etc.), and store a plurality of application programs or applications that are driven on the computer device 100, and data and commands for the operation of the computer device 100. At least some of the application programs may be downloaded from an external server via wireless communication.

Such memory 110 may include at least one type of storage medium among a flash memory type, a hard disk type, a solid state disk type (SSD type), a silicon disk drive type (SDD type), a multimedia card micro type, a card-type memory (e.g., an SD or XD memory), a random access memory (RAM), a static random access memory (SRAM), a read-only memory (ROM), an electrically erasable programmable ROM (EEPROM), a programmable ROM (PROM), a magnetic memory, a magnetic disk, and an optical disk. In addition, the memory may be a database that is separate from the device but connected in a wired or wireless connection manner.

Referring to FIG. 2, the processor 120 may control all components in the computer device 100 to process signals, data, information, etc. that are input or output, or perform various processes by executing commands, algorithms, and application programs that are stored in the memory 110, and provide or process appropriate information or functions to each user by implementing a data augmentation procedure. The components shown in FIG. 2 are not essential for implementing the computer device 100 according to the present disclosure, and the computer device 100 described in the present disclosure may include more or fewer components than the components listed above. In this case, the computer device 100 may refer to a data augmentation device.

The processor 120 may communicate with the memory 110 and implement augmentation of original data to be trained.

The processor 120 may select a plurality of the augmentation target data including first-type data and second-type data to be augmented according to a predetermined selection condition from the original data. In this case, the first-type data may refer to positive data, and the second-type data may refer to negative data.

The original data may be the peptide feature for binding of the MHC class II feature. In this case, the original data may include whether each peptide feature is bound to the MHC class II and its immunogenicity. Based on this information, the processor 120 selects the augmentation target data according to the predetermined selection condition.

The selection condition may include a predetermined IC50 value, i.e., a concentration value, and a predetermined number of a peptide length.

For example, referring to FIG. 3, when selecting the plurality of augmentation target data, the processor 120 may select the first-type data including at least one positive data matching a first selection condition among the original data.

The first selection condition may be a condition in which an IC50 label is less than the predetermined concentration value and the peptide length is less than or equal to the predetermined number. For example, the first selection condition may be a condition in which the IC50 label is greater than 0.01 nM and less than 500 nM (0.01 nM < IC50 label < 500 nM), and the peptide length is greater than or equal to 10 and less than 20 (10 ≤ peptide length < 20).

In this case, a half maximal inhibitory concentration(IC)50 may refer to the maximal concentration at which the activity of a cell (enzyme/protein activity) is reduced by half when a drug is administered. In this case, the indicator representing the activity of the cell may be a protein. A smaller IC50 value may indicate higher affinity.

That is, the selected first-type data may be a positive subset composed of a plurality of positive data, a qualitative label is positive high, a quantitative label is the IC50 greater than 0.01 nM and less than 500 nM, and the peptide length is greater than or equal to 10 and less than 20. Since structural deformation may occur when the peptide length is too long, the peptide length may be set to be restricted.

As another example, referring to FIG. 3, when selecting the plurality of augmentation target data, the processor 120 may select the second-type data including at least one negative data matching a second selection condition among the original data.

The second selection condition may be a condition in which the IC50 label is greater than the predetermined concentration value and the peptide length is greater than or equal to the predetermined number. For example, the second selection condition may be a condition in which the IC50 label is greater than 50000 nM and less than 5000000 nM (50000 nM < IC50 label < 5000000 nM), and the peptide length is greater than 11 and less than or equal to 30 (11 < peptide length ≤ 30).

That is, the selected second-type data may be a negative subset composed of a plurality of negative data, the qualitative label is negative, the quantitative label is the IC50 greater than 50000 nM and less than 5000000 nM, and the peptide length is greater than 11 and less than or equal to 30.

The processor 120 may augment the selected plurality of augmentation target data according to a predetermined augmentation condition, by respectively augmenting the first-type data and the second-type data according to an augmentation condition of the first-type data and an augmentation condition of the second-type data, to generate a plurality of augmentation data.

The augmentation condition of the first-type data may include a condition in which random augmentation is applied to the positive subset and a condition in which a human protein pattern is applied. In addition, the augmentation condition of the second-type data may be a condition in which the length is shortened by removing the peptide sequence of the negative subset.

As an example, when generating the plurality of augmentation data, the processor 120 may randomly add all amino acids to each of the plurality of augmentation target data, as follows. In this case, the processor 120 may randomly add all amino acids except cystein as a sequence.

Specifically, referring to FIG. 4, the processor 120 may add a randomly selected amino acid sequence to an N-terminus of a peptide original sequence of the first-type data as one sequence, A1. In addition, the processor 120 may add a randomly selected amino acid sequence to a C-terminus of the peptide original sequence of the first-type data as one sequence, A2. In addition, the processor 120 may add a randomly selected amino acid sequence to each of the N-terminus and the C-terminus of the peptide original sequence of the first-type data as one sequence, A3-1 and A3-2. The processor 120 may augment the first-type data of the plurality of augmentation target data by the above-described method.

In this case, the number of peptide original sequences shown in FIG. 4 is merely an example for explanation and may be considered to include a binding core sequence.

As another example, when generating the plurality of augmentation data, the processor 120 may add a sequence to each of the plurality of augmentation target data using an amino acid sequence pattern(4-mer) of human proteins, as follows.

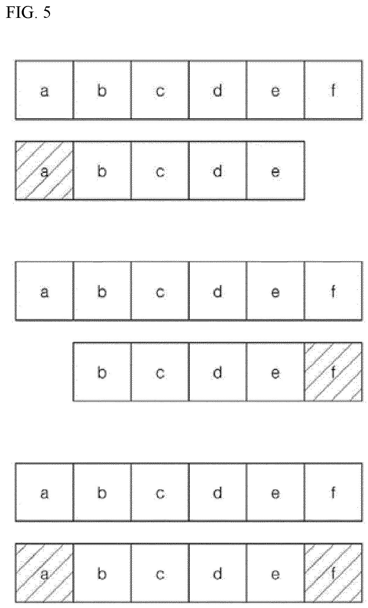

Specifically, referring to FIG. 5, the processor 120 may add one sequence (a) to the N-terminus of the peptide original sequence of the first-type data using the amino acid sequence pattern (a, b, c, d, e, d f) of the human proteins. The processor 120 may add one sequence (f) to the C-terminus of the peptide original sequence of the first-type data using the amino acid sequence pattern of the human proteins. The processor 120 may add one sequence (a, f) to each of the N-terminus and C-terminus of the peptide original sequence of the first-type data using the amino acid sequence pattern of the human proteins. The processor 120 may augment the first-type data of the plurality of augmentation target data by the above-described method.

For example, the processor 120 may add the sequences to the N-terminus and C-terminus using the sequence patterns(4-mer) that appear more than 100 times in the human proteins. The present embodiment may be expected to have an effect of enabling data augmentation similar to reality. In this case, the number of peptide original sequences shown in FIG. 5 is merely an example for explanation and may be considered to include the binding core sequence.

As another example, referring to FIG. 6, when generating the plurality of augmentation data, the processor 120 may remove sequences D6-1 and D6-2 from both termini of a peptide original sequence of the second-type data in the plurality of augmentation target data until a length of the peptide original sequence matches a predetermined number of sequences (N). In this case, the processor 120 may generate additional augmentation data each time the processor 120 removes both termini of the peptide original sequence one by one.

The processor 120 may be implemented to modify labeling of the plurality of augmentation data so that labels are modified according to different labeling conditions for the first-type data and the second-type data.

When modifying the labeling of the plurality of augmentation data, the processor 120 may normalize the label of the corresponding original data of each of the plurality of augmentation data, and obtain a final pseudo label according to the labeling conditions for the first-type data and the second-type data. In this case, modifying to the pseudo label is a technique that assigns the most probable label in the form of a virtual label, which may be a method to overcome the limitation of data with insufficient label values.

The final pseudo label may be calculated using a predetermined label constant value based on the normalized pseudo label of the original data and the binding affinity of the peptide to the MHC class II molecule.

Specifically, referring to FIG. 7, the processor 120 may normalize the label(original label) of the corresponding original data of the augmentation data to a range from 0 to 1 for convenience, and then process the normalized label to modify to the final pseudo label. In this case, the label of the corresponding original data of the augmentation data may refer to the label of the original data (the augmentation target data) before augmentation of the augmentation data.

The above-described 0 may indicate low affinity or low immunogenicity, and 1 may indicate high affinity or high immunogenicity.

When the augmentation data is the positive subset that is the first-type data, the processor 120 may calculate the final pseudo label(pseudo label > x - k) as a value greater than the value obtained by subtracting a predetermined label constant value k from the normalized pseudo label (x) of the original data. In this case, the predetermined label constant value k may be determined as the constant with the highest peptide binding affinity performance obtained through model training for the MHC class II molecule, which may vary for each task. For example, in the case of binding affinity, the predetermined label constant value k may be 0.25, and in the case of immunogenicity, the predetermined label constant value k may be 0.15.

When the augmentation data is the negative subset that is the second-type data, the processor 120 may calculate the final pseudo label(pseudo label < x - k) as a value less than the value obtained by subtracting the predetermined label constant value k from the normalized pseudo label (x) of the original data.

That is, the processor 120 may modify the label of the original data to a range between 0 and 1 using 1 - log(IC50)/log50000, where 1 corresponds to high affinity, and proceed with the subsequent procedures. Although the IC50 value of the present disclosure indicates higher affinity with smaller values, the normalized label may indicate higher affinity with larger values.

The processor 120 may delete duplicate data by comparing the plurality of augmentation data and the original data.

For example, when both the original data and the augmentation data are A, the processor 120 may delete one of them to prevent noise and unnecessary training procedure in advance.

The processor 120 may generate a validation set to verify a fair learning model and perform a validation procedure. In this case, the validation set may be composed only of the original data without performing augmentation.

Meanwhile, the computer device 100 may include one or more components that enable communication with an external device and may include, for example, the communication interface 130 for wireless communication and an input/output interface 140 for wired communication.

Specifically, the communication interface 130 may transmit and receive signals to and from the external device via a network 200 based on wireless communication. To this end, the communication interface 130 may include at least one wireless communication module, short range communication module, and the like.

First, the wireless communication module may include wireless communication modules that support various wireless communication methods such as global system for mobile communication (GSM), code division multiple access (CDMA), wideband code division multiple access (WCDMA), universal mobile telecommunications system (UMTS), time division multiple access (TDMA), wireless local area network (WLAN), digital living network alliance (DLNA), wireless broadband (WiBro), worldwide interoperability for microwave access (WiMAX), high-speed downlink packet access (HSDPA), high-speed uplink packet access (HSUPA), long-term evolution (LTE), 4G, 5G, and 6G in addition to a Wi-Fi module and a wireless broadband module.

In addition, the short range communication module is configured for short range communication and may support short range communication using at least one of Bluetooth^{™}, radio frequency identification (RFID), infrared data association (IrDA), ultra wideband (UWB), ZigBee, near field communication (NFC), wireless-fidelity (Wi-Fi), Wi-Fi direct, and wireless universal serial bus (Wireless USB) techniques.

The input/output interface 140 may be connected to the input/output devices 150 in a wired manner, that is, based on wired communication, to transmit and receive signals. To this end, the input/output interface 140 may include at least one wired communication module, and the wired communication module may include not only various wired communication modules such as a local area network (LAN) module, a wide area network (WAN) module, or a value-added network (VAN) module, but also various cable communication modules such as universal serial bus (USB), high definition multimedia interface (HDMI), digital visual interface (DVI), recommended standard 232 (RS-232), powerline communication, or plain old telephone service (POTS).

Although not shown, the data augmentation device 100 of the present disclosure may further include an output unit and an input unit.

The output unit may display a user interface (UI) for providing data augmentation results or the like. The output unit may output any form of information generated or determined by the processor 120 and any form of information received by the communication interface 130.

The output unit may include at least one of a liquid crystal display (LCD), a thin film transistor-liquid crystal display (TFT LCD), an organic light-emitting diode (OLED), a flexible display, and a 3D display. Some of these display modules may be configured as a transparent type or a light-transmitting type to allow external visibility through them. This may be referred to as a transparent display module, and a representative example of the transparent display module includes a transparent OLED (TOLED) or the like.

The input unit may receive information input by a user. The input unit may include keys and/or buttons on a user interface, or physical keys and/or buttons, for receiving information input by a user. A computer program for controlling the display according to the embodiments of the present disclosure based on user input through the input unit may be executed.

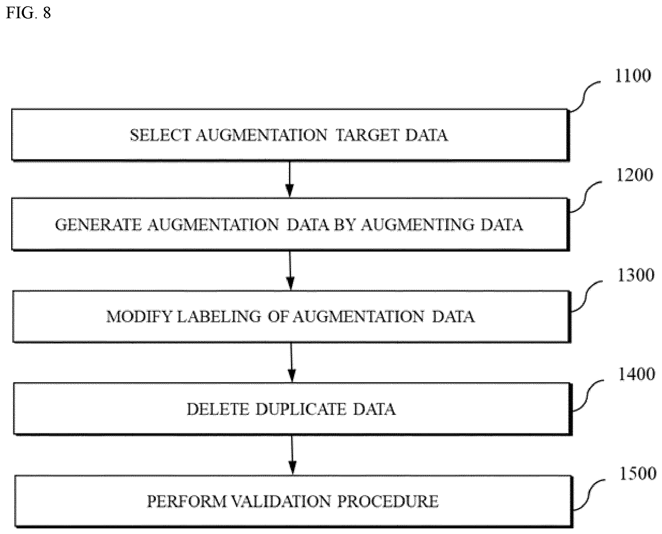

FIG. 8 is a flowchart for describing a data augmentation method according to the present disclosure.

The processor 120 of the computer device 100 may select a plurality of augmentation target data including first-type data and second-type data to be augmented according to a predetermined selection condition from original data (1100). In this case, the first-type data may refer to positive data, and the second-type data may refer to negative data. The original data may be a peptide feature for binding of a major histocompatibility complex (MHC) class II feature.

Next, the processor 120 may augment the selected plurality of augmentation target data according to a predetermined augmentation condition, by respectively augmenting the first-type data and the second-type data according to the augmentation condition of the first-type data and the augmentation condition of the second-type data, to generate a plurality of augmentation data (1200).

Next, the processor 120 may modify labeling of the plurality of augmentation data so that labels are modified according to different labeling conditions for the first-type data and the second-type data (1300).

When modifying the labeling of the plurality of augmentation data, the processor 120 may normalize the label of the corresponding original data of each of the plurality of augmentation data, and obtain a final pseudo label according to the labeling conditions for the first-type data and the second-type data. The final pseudo label may be calculated using a predetermined label constant value based on the normalized pseudo label of the original data and a binding affinity of a peptide to a MHC class II molecule.

Next, the processor 120 may delete duplicate data by comparing the plurality of augmentation data with the original data (1400).

The processor 120 may generate a validation set to verify a fair learning model and perform a validation procedure (1500). In this case, the validation set may be composed only of the original data without performing augmentation.

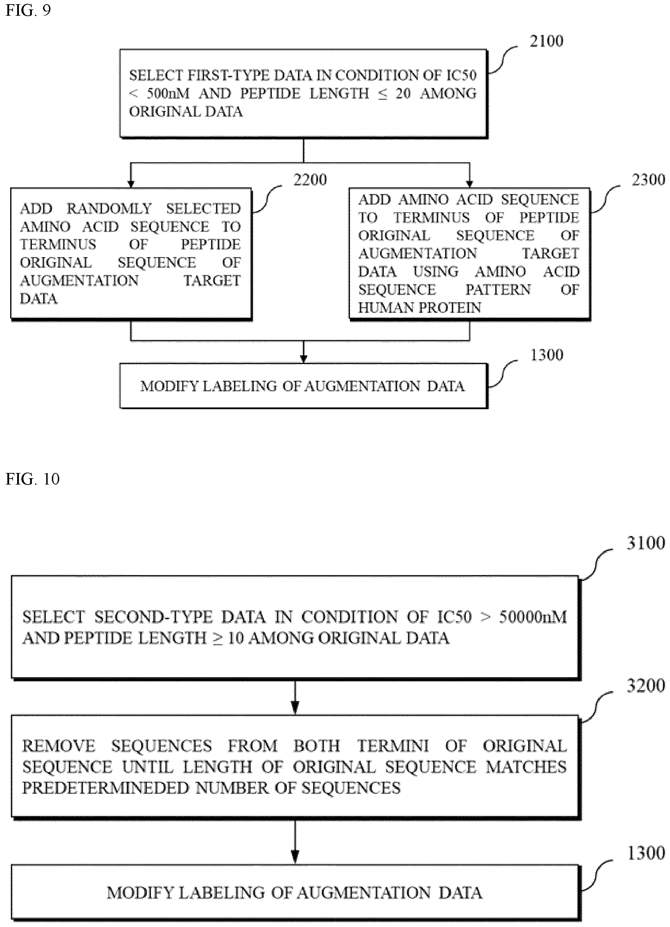

FIGS. 9 is a flowchart for describing the data augmentation method of FIG. 8 in detail, and a method of augmenting the augmentation target data of the first-type data will be described as an example.

First, when selecting the plurality of augmentation target data, the processor 120 may select the first-type data including at least one positive data matching a first selection condition among the original data (2100).

The first selection condition may be a condition in which an IC50 label is less than a predetermined concentration value and a peptide length is less than or equal to a predetermined number. For example, the first selection condition may be a condition in which the IC50 label is greater than 0.01 nM and less than 500 nM (0.01 nM < IC50 label < 500 nM), and the peptide length is greater than or equal to 10 and less than 20 (10 ≤ peptide length < 20).

Next, when generating the plurality of augmentation data, the processor 120 may randomly add all amino acids to each of the plurality of augmentation target data, as follows (2200). In this case, the processor 120 may randomly add all amino acids except cystein as a sequence.

Specifically, referring to FIG. 4, the processor 120 may add a randomly selected amino acid sequence to an N-terminus of a peptide original sequence of the first-type data as one sequence. In addition, the processor 120 may add a randomly selected amino acid sequence to a C-terminus of the peptide original sequence of the first-type data as one sequence. In addition, the processor 120 may add a randomly selected amino acid sequence to each of the N-terminus and C-terminus of the peptide original sequence of the first-type data as one sequence. The processor 120 may augment the first-type data of the plurality of augmentation target data by the above-described method.

In this case, the number of peptide original sequences shown in FIG. 4 is merely an example for explanation and may be considered to include a binding core sequence.

As another example, when generating the plurality of augmentation data, the processor 120 may add a sequence to each of the plurality of augmentation target data using an amino acid sequence pattern(4-mer) of human proteins, as follows (2300).

Specifically, referring to FIG. 5, the processor 120 may add one sequence (a) to the N-terminus of the peptide original sequence of the first-type data using the amino acid sequence pattern of the human proteins. The processor 120 may add one sequence (f) to the C-terminus of the peptide original sequence of the first-type data using the amino acid sequence pattern of the human proteins. The processor 120 may add one sequence (a, f) to each of the N-terminus and C-terminus of the peptide original sequence of the first-type data using the amino acid sequence pattern of the human proteins. The processor 120 may augment the first-type data of the plurality of augmentation target data by the above-described method.

For example, the processor 120 may add the sequences to the N-terminus and C-terminus using the sequence patterns(4-mer) that appear more than 100 times in the human proteins. The present embodiment may be expected to have an effect of enabling data augmentation similar to reality. In this case, the number of peptide original sequences shown in FIG. 5 is merely an example for explanation and may be considered to include the binding core sequence.

Next, the processor 120 may perform operations after operation 1300 of FIG. 8.

FIGS. 10 is a flowchart for describing the data augmentation method of FIG. 8 in detail, and a method of augmenting the augmentation target data of the second-type data will be described as an example.

Referring to FIG. 10, when selecting the plurality of augmentation target data, the processor 120 may select second-type data including at least one negative data matching a second selection condition among the original data (3100).

The second selection condition may be a condition in which the IC50 label is greater than a predetermined concentration value and the peptide length is greater than or equal to a predetermined number. For example, the second selection condition may be a condition in which the IC50 label is greater than 50000 nM and less than 5000000 nM (50000 nM < IC50 label < 5000000 nM), and the peptide length is greater than 11 and less than or equal to 30 (11 < peptide length ≤ 30).

Next, referring to FIG. 6, when generating the plurality of augmentation data, the processor 120 may remove sequences from both termini of a peptide original sequence of the second-type data in the plurality of augmentation target data until a length of the peptide original sequence matches a predetermined number of sequences (N) (3200). In this case, the processor 120 may generate additional augmentation data each time the processor 120 removes both termini of the peptide original sequence one by one.

Next, the processor 120 may perform operations after operation 1300 of FIG. 8.

Meanwhile, the above-described method according to the present disclosure may be implemented as a program (or application) to be executed in conjunction with hardware such as a server and stored in a medium.

The disclosed embodiments may be implemented in the form of a recording medium in which computer-executable commands are stored. The commands may be stored in the form of program code, and when executed by the processor, program modules are generated to perform operations of the disclosed embodiments. The recording medium may be implemented as a computer-readable recording medium.

The computer-readable recording medium includes all types of recording media in which computer-decodable commands are stored. For example, there may be a read only memory(ROM), a random access memory(RAM), a magnetic tape, a magnetic disk, a flash memory, an optical data storage device, and the like.

As described above, the disclosed embodiments have been described with reference to the accompanying drawings. Those skilled in the art to which the present disclosure pertains will understand that the present disclosure may be implemented in different forms from the disclosed embodiments without departing from the technical spirit or essential features of the present disclosure. The disclosed embodiments are illustrative and should not be construed as being limited.

## Claims

1. A data augmentation device, comprising:
a memory; and
a processor configured to communicate with the memory and implement augmentation of original data to be trained,
wherein
the processor is implemented to:
select a plurality of augmentation target data including first-type data and second-type data to be augmented according to a predetermined selection condition from the original data;
augment the selected plurality of augmentation target data according to a predetermined augmentation condition, wherein the first-type data and the second-type data are respectively augmented according to an augmentation condition of the first-type data and an augmentation condition of the second-type data to generate a plurality of augmentation data; and
modify labeling of the plurality of augmentation data, wherein labels are modified according to different labeling conditions for the first-type data and the second-type data, and
the original data is a peptide feature for binding of a major histocompatibility complex (MHC) class II feature.

2. The data augmentation device of claim 1, wherein
when selecting the plurality of augmentation target data,
the processor selects the first-type data including at least one positive data matching a first selection condition among the original data, wherein the first selection condition is a condition in which an IC50 label is less than a predetermined concentration value and a peptide length is less than or equal to a predetermined number.

3. The data augmentation device of claim 1, wherein
when selecting the plurality of augmentation target data,
the processor selects the second-type data including at least one negative data matching a second selection condition among the original data, wherein the second selection condition is a condition in which an IC50 label is greater than a predetermined concentration value and a peptide length is greater than or equal to a predetermined number.

4. The data augmentation device of claim 1, wherein
when generating the plurality of augmentation data,
the processor randomly adds all amino acids to each of the plurality of augmentation target data, wherein a randomly selected amino acid sequence is added to an N-terminus of a peptide original sequence of the first-type data as one sequence, a randomly selected amino acid sequence is added to a C-terminus of the peptide original sequence of the first-type data as one sequence, and a randomly selected amino acid sequence is added to each of the N-terminus and C-terminus of the peptide original sequence of the first-type data as one sequence, to augment the first-type data of the plurality of augmentation data.

5. The data augmentation device of claim 1, wherein
when generating the plurality of augmentation data,
the processor adds a sequence to each of the plurality of augmentation target data using an amino acid sequence pattern of a human protein, wherein one sequence is added to an N-terminus of a peptide original sequence of the first-type data using the amino acid sequence pattern of the human protein, one sequence is added to a C-terminus of the peptide original sequence of the first-type data using the amino acid sequence pattern of the human protein, and one sequence is added to each of the N-terminus and C-terminus of the peptide original sequence of the first-type data using the amino acid sequence pattern of the human protein, to augment the first-type data of the plurality of augmentation data.

6. The data augmentation device of claim 1, wherein
when generating the plurality of augmentation data,
the processor removes sequences from both termini of a peptide original sequence of the second-type data in the plurality of augmentation target data until a length of the peptide original sequence matches a predetermined number of sequences.

7. The data augmentation device of claim 1, wherein
when modifying the labeling of the plurality of augmentation data,
the processor normalizes a label of the corresponding original data of each of the plurality of augmentation data, and obtains a final pseudo label according to the labeling conditions for the first-type data and the second-type data, wherein the final pseudo label is calculated using a predetermined label constant value based on the normalized pseudo label of the original data and a binding affinity of a peptide to an MHC class II molecule.

8. The data augmentation device of claim 1, wherein
the processor deletes duplicate data by comparing the plurality of augmentation data with the original data.

9. A data augmentation method performed by a computer device, the method comprising:
selecting a plurality of augmentation target data including first-type data and second-type data to be augmented according to a predetermined selection condition from original data,
augmenting the selected plurality of augmentation target data according to a predetermined augmentation condition, wherein the first-type data and the second-type data are respectively augmented according to an augmentation condition of the first-type data and an augmentation condition of the second-type data to generate a plurality of augmentation data; and
modifying labeling of the plurality of augmentation data, wherein labels are modified according to different labeling conditions for the first-type data and the second-type data,
wherein
the original data is a peptide feature for binding of a major histocompatibility complex (MHC) class II feature.

10. The method of claim 9, wherein
when selecting the plurality of augmentation target data,
the method selects the first-type data including at least one positive data matching a first selection condition among the original data, wherein the first selection condition is a condition in which an IC50 label is less than a predetermined concentration value and a peptide length is less than or equal to a predetermined number.

11. The method of claim 9, wherein
when selecting the plurality of augmentation target data,
the method selects the second-type data including at least one negative data matching a second selection condition among the original data, wherein the second selection condition is a condition in which an IC50 label is greater than a predetermined concentration value and a peptide length is greater than or equal to a predetermined number.

12. The method of claim 9, wherein
when generating the plurality of augmentation data,
the method randomly adds all amino acids to each of the plurality of augmentation target data, wherein a randomly selected amino acid sequence is added to an N-terminus of a peptide original sequence of the first-type data as one sequence, a randomly selected amino acid sequence is added to a C-terminus of the peptide original sequence of the first-type data as one sequence, and a randomly selected amino acid sequence is added to each of the N-terminus and C-terminus of the peptide original sequence of the first-type data as one sequence, to augment the first-type data of the plurality of augmentation data.

13. The method of claim 9, wherein
when generating the plurality of augmentation data,
the method adds a sequence to each of the plurality of augmentation target data using an amino acid sequence pattern of a human protein, wherein one sequence is added to an N-terminus of a peptide original sequence of the first-type data using the amino acid sequence pattern of the human protein, one sequence is added to a C-terminus of the peptide original sequence of the first-type data using the amino acid sequence pattern of the human protein, and one sequence is added to each of the N-terminus and C-terminus of the peptide original sequence of the first-type data using the amino acid sequence pattern of the human protein, to augment the first-type data of the plurality of augmentation data.

14. The method of claim 9, wherein
when generating the plurality of augmentation data,
the method removes sequences of both termini of a peptide original sequence of the second-type data in the plurality of augmentation target data until a length of the peptide original sequence matches a predetermined number of sequences.

15. The method of claim 9, wherein
when modifying the labeling of the plurality of augmentation data,
the method normalizes a label of the corresponding original data of each of the plurality of augmentation data, and obtains a final pseudo label according to the labeling conditions for the first-type data and the second-type data, wherein the final pseudo label is calculated using a predetermined label constant value based on the normalized pseudo label of the original data and a binding affinity of a peptide to an MHC class II molecule.

16. The method of claim 9, wherein
after modifying labeling of the plurality of augmentation data,
the method deletes duplicate data by comparing the plurality of augmentation data with the original data.

17. A program stored in a computer-readable recording medium to execute the data augmentation method of any one of claims 9 to 16 in conjunction with a computer.
